# EUROPEAN PATENT SPECIFICATION

(11) **EP 1 755 437 B1**
(45) Date of publication and mention of the grant of the patent: **08.01.2014**
(21) Application number: 05750336.9
(22) Date of filing: 06.06.2005
(51) Int. Cl.: A61B 1/015

(54) **MULTIPURPOSE ENDOSCOPY SUITE**
MEHRZWECK-ENDOSKOPIEAUSRÜSTUNG
ÉQUIPEMENT POUR ENDOSCOPIE À USAGES MULTIPLES

(30) Priority: 07.06.2004 IL 16239004
(43) Date of publication of application: 28.02.2007
(73) Proprietor: Medigus Ltd., 84965 Omer (IL)
(72) Inventor: SONNENSCHEIN, Elazar, 84800 Beer Sheva (IL); ELOVICI, Yuval, 79280 Moshav Shetolim (IL); SONNENSCHEIN, Minelu, 85025 Meitar (IL)
(74) Representative: Tiesmeyer, Johannes
(86) International application number: PCT/IL2005/000598
(87) International publication number: WO 2005/120329

(56) References cited:
- US-A- 5 788 688
- US-A- 5 931 808
- US-A- 6 092 722
- US-A1- 2004 064 019
- US-A1- 2004 095 507

## Description

### Field of the Invention

The invention relates to the field of endoscopy suites. More particularly, the invention relates to a multipurpose endoscopy suite to which different types of endoscopes may be connected for carrying out various types of endoscopy procedures.

### Background of the Invention

Endoscopes are in general rigid, semiflexible, or flexible instruments that are used to examine interior organs and to carry out various medical procedures inside a patient's body without the necessity of performing an open major surgery. A flexible endoscope usually consists of a control handle and an insertion tube having a manoeuvrable tip. On the tip are located various elements such as a lens for an imaging system, an ultrasound transducer, irrigation nozzle, a miniature camera, stapler, etc. Channels extend through the interior of the endoscope from the distal tip to the proximal end. Typically these channels are of two types: (1) working channels to allow the surgeon to employ medical instruments relevant to carrying out specific tasks during the procedure, e.g., biopsy forceps, other small diameter endoscopes, tools such as scissors, small diameter staplers or suture device, (2) tubes that are necessary for delivering air and irrigation, cables that carry the imaging system signals (e.g., CCD or CMOS signals), cables that carry the ultrasound signals, fibers that carry the illumination toward the distal tip from a proximal light source, and often suction and insufflation channels. Special controls that are normally connected to cables that also pass through the interior channels allow the operator to maneuver the tip of the endoscope through interior passages of the body and perform complex procedures.

Endoscopy has advanced in recent years to the stage where many different procedures which are related to a specific body system or organ have become commonplace and are carried out in large numbers. Examples of some of these procedures are: Colonoscopy to examine the lining of the colon; Gastroscopy (EGD), carried out through the esophagus to view the stomach and upper gastroenterological tract; ERCP (Endoscopic Retrograde Cholangiopancreatography) for examining and treating the liver, gallbladder, bile ducts and pancreas; Bronchoscopy for viewing the breathing passages; and Transgastric or Extragastric procedures in the abdomen. In Transgastric procedures a mother scope including two or more small diameter baby scopes is advanced into the stomach and is used to view the stomach. The stomach tissue is pierced with a tool that is extended from the working channel of the mother scope and then the distal tip of the motherscope is advanced through the incision into the abdomen where a procedure is performed using the babyscopes. If necessary the abdomen is inflated with air or Co2 gas. Transgastric procedures may include procedures such as Appendectomy, Cholecystectomy, Liver biopsy and excision, Gastric banding for morbid obesity, and implanting of medical devices, for example a device for tubal ligation or devices to be anchored on internal organs. Hence the endoscopy suite will need to support more than one endoscope, several pumps, various types of gas, and various types of optical, imaging, measurement, and control systems.

Each procedure has different requirements that are dictated by factors such as the diameter, curvature and complexity, of the passageways of the body through which the endoscope must be inserted. For example, the endoscope used in Gastroscopy procedures is typically designed for diameters of about 8-14mm, while endoscopes used for carrying out bronchoscopy procedures are designed for passageways which are less than 6mm in diameter. Another example is Transgastric procedures. The endoscope used in a procedure for tubal ligation or Cholecystectomy is typically designed with a diameter of about 14-16mm and with three or more working channels. Two of the working channels contain small diameter endoscopes (baby scopes), each having a diameter of 3.9mm. Each baby scope contains a working channel of 1.2mm. In another option the endoscope comprises two 2.5mm diameter baby scopes that use an imager of "1/15". A stapling or suturing device is contained in a third channel or as a part of the mother scope. As can be understood from the above, the differing requirements of different procedures have resulted in the development of a large variety of specialized endoscopes.

The peripheral equipment and resources referred to hereinabove, e.g. illumination, irrigation, etc. required for operating the various types of procedure oriented endoscopes is essentially the same, however considerations such as the amount of illumination that must be supplied or the maximum amount of suction/insufflation/irrigation that can be safely applied vary from procedure to procedure. The peripheral equipment is packaged in units known as endoscopy suites and historically there has arisen a situation wherein each type of endoscope and/or endoscopic procedure comes together with a dedicated endoscopy suite.

The main advantage in the provision of endoscopy suites specifically suitable for carrying out a certain procedure is in the simplicity of its operation and in its safety. This is because the operator does not have to be concerned with the settings of the different procedure-related parameters (e.g., light intensity, insufflation and suction pressures, etc) since these are determined in advance, set during the production stage of the endoscopy suite, and cannot be easily changed. The major disadvantage is that hospitals and clinics at which many different types of endoscopic procedures are routinely carried out must maintain a supply of endoscopy suites, often several in a single operating theater. In addition, the cost of a procedure oriented endoscopy suite is often linked to the particular medical procedure with which it is associated and not entirely to the actual manufacturing costs, thus the present practice of supplying a dedicated endoscopy suite for every type of procedure and endoscope leads to inefficient use of resources and ultimately to inflated cost of the medical procedure.

In view of the fact that most endoscopy procedures require more or less the same peripheral equipment and resources, and the main differences between different endoscopic procedures is the range of parameters within which the equipment must work, it would be advantageous to supply a single endoscopy suite that is capable of being used with many, if not all, types of endoscopes.

It is an object of the present invention to provide a multipurpose endoscope suite that can be adapted for carrying out a plurality of endoscopy procedures.

It is another object of the present invention to provide a multipurpose endoscope suite for which the operator can select and or adjust the values of the operating parameters making them suitable for a particular endoscopy procedure.

It is still another object of the present invention to provide an endoscope suite that can be easily calibrated utilizing programmable means through it's input keypad.

It is still another object of the present invention to provide endoscopes suite that can simultaneously support more than two different types of endoscopes used during a single procedure.

Other objects and advantages of the invention will become apparent as the description proceeds.

US 5, 931, 808 teaches an endoscopy suite which, inter alia, has no ultrasound components and no programmable resistors in analog cut off circuits.

### Summary of the Invention

The present invention is defined in claim 1 and provides an endoscopy suite that is adaptable for carrying out a plurality of endoscopy procedures and/or for supporting several endoscopes being used simultaneously in a given procedure.

The endoscopy suite of the invention comprises one or more lamps adapted for providing light (generally white light) that can be conducted to the distal end of the endoscope/s. It also comprises one or more fans for cooling the lamp/s, additional power sources, which may include ballast means, for operating the lamp/s. The lamp/s may be selected from the group comprising: xenon lamps, halogen lamps, and metal halide lamps. One or more beam splitters can be provided to allow the light from one lamp to be used to supply light to two or more illumination channels. Other means of light can be used to illuminate the region in which the procedure is to be carried out. For example, light-emitting diodes (LEDs) can be placed on the distal tip of the endoscope in which case the endoscopy suite will comprise a power source that will be connected to the LEDs by means of electrical cables. In another option the light from LEDs can be transmitted by fibers up to the distal tip of the endoscope. The endoscopy suite of the invention may comprise means linked to the memory for adjusting the amount of light delivered from the lamp according to a predetermined value selected from one or more predetermined values stored in the memory and selected by an input received from the input device. The means for controlling the amount of light delivered from the lamp may comprise an iris attached to a motor adapted to respond to signals for adjusting the opening of the iris. The motor can be a step motor. An optical sensor may be provided to sense the home position of the stepping motor.

The endoscopy suite of the invention comprises an ultrasound module for generating ultrasound signals to be emitted by an ultrasound transducer on the endoscope, detecting and processing ultrasound signals received by the transducer, and outputting the processed signals to a display device. The endoscopy suite comprises an input device linked to the ultrasound module for modifying the operation and settings of the module. The ultrasound signals can be used to measure distances, position, or tissue thickness within the body.

The endoscopy suite of the invention comprises a video module for acquiring video signals from one or more cameras on the endoscope/s, processing the signals, and outputting the processed signals to a display device. The video module may be able to receive signals from several camera heads on the same or on several different endoscopes and display the signals received properly on one or several display devices. The endoscopy suite comprises an input device linked to the video module for modifying the operation and settings of the module. The video module can be adapted to receive output signals form the ultrasound module and to store them in a memory, to transmit them to the network, to display them along with the output video signals on a display, and/or to transmit them to a printer.

The video and ultrasound modules can be housed in a unit having its own power source and the remainder of the components of the endoscopy suite can be housed in another unit. Each of the units may comprise one or more fans for cooling the components housed within it. The fans and other electrical components are controlled by a central unit so in case of fan failure it is possible to advise the operator to stop the procedure.

The endoscopy suite of the invention comprises a processor capable of managing the data stored in the memory, receiving inputs from the input device, and outputting signals to a display device and to the circuitry controlling the operation of the pumps. The endoscopy suite comprises a communication interface linked to the processor and a communication interface linked to the ultrasound module and a communication interface linked to the video module and optionally a communication interface linked to a video printer.

The circuitry controlling the pumps of the endoscopy suite of the invention comprises cut off circuitries and programmable resistors adapted to receive predetermined values from the memory. The predetermined parameters for each pump include a value of low pressure at which the pump is activated and a value of high pressure at which the pump is deactivated.

The preferred embodiment of the endoscopy suite of the invention comprises means for controlling and monitoring the status of surgical tools, e.g. a surgical stapler used to perform endoscopic procedures.

The endoscopy suite of the invention can be used to perform an endoscopy procedure selected from the group comprising: a Gastroscopy procedure; an ERCP procedure using a mother scope and a baby scope; a Colonoscopy procedure; a Gynecology procedure; a Bronchoscopy procedure; an ENT procedure; an Endoluminal anti Reflux procedure; a Transgastric or Extragastric procedure using a mother scope and one or more baby scopes; and an Anti Reflux proceedure. The Transgastric or Extragastric procedure can be selected from the group comprising: Appendectomy; Cholecystectomy; liver biopsy and excision; gastric banding for morbid obesity; and implantation of medical devices.

All the above and other characteristics and advantages of the invention will be further understood through the following illustrative and non-limitative description of preferred embodiments thereof, with reference to the appended drawings.

### Brief Description of the Drawings

In the drawings:
- Fig. 1 is a block diagram illustrating a preferred embodiment of a unit for suction, insufflations, and light (ISL) intensity control according to the invention;
- Fig. 2 is a block diagram illustrating a preferred ultrasound and video control unit according to a preferred embodiment of the invention;
- Fig. 3 illustrates an embodiment of a user interface for the endoscopy suite of the invention; and
- Fig. 4 is a block diagram illustrating the ISL board in a preferred embodiment of the invention.

### Detailed Description of Preferred Embodiments

The present invention is directed to a multipurpose endoscopy suite that can be used for carrying out different endoscopy or transgastric procedures. The endoscopy suite of the invention is designed to comprise reconfigurable equipment and resources that can be used for carrying out different procedures at different times according to user defined or, preferably, predetermined sets of parameters that are particularly suitable for the specific procedure.

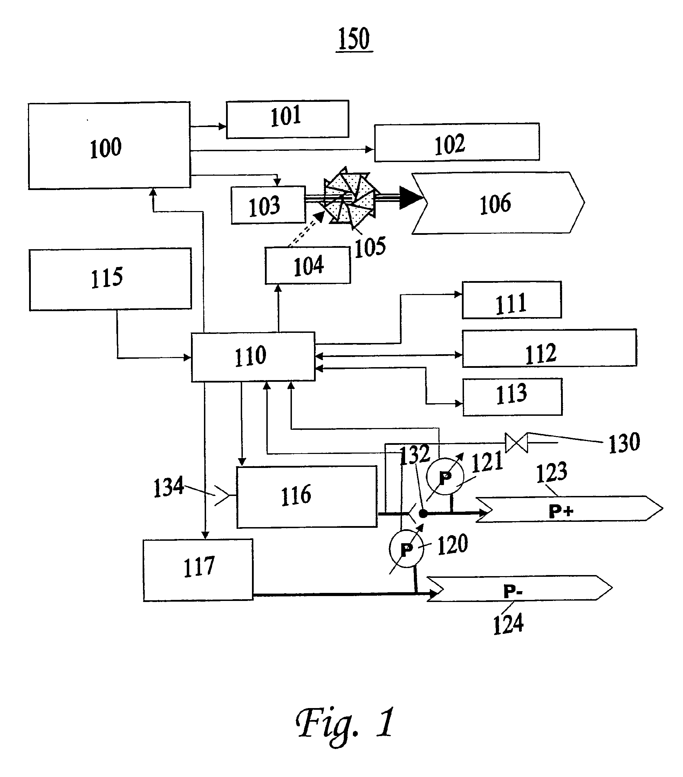

Fig. 1 is a block diagram showing a ISL system for providing insufflation, suction, and light intensity (ISL) control. As has been discussed hereinabove, various procedures that are carried out require the simultaneous use of two or more endoscopes. Preferred embodiments of the endoscopy suite of the invention are designed to provide the requirements of several endoscopes simultaneously. In order to simplify the description of the invention, the system shown in the figures and described hereinbelow relates to an ISL system for attachment to a single endoscope only. The description provided herein along with the general knowledge possessed by skilled persons will suffice to allow the design of similar systems capable of being used with multiple endoscopes.

The ISL system is preferably enclosed in an independent unit (Console) 150. The unit 150 preferably comprises two independent power sources: a light source power supply 100 for supplying power to lamp 103, lamp fans 101, and general fan 102 (used to cool the interior of unit 150); and an ISL board power supply 115, which provides power to the ISL board 110.

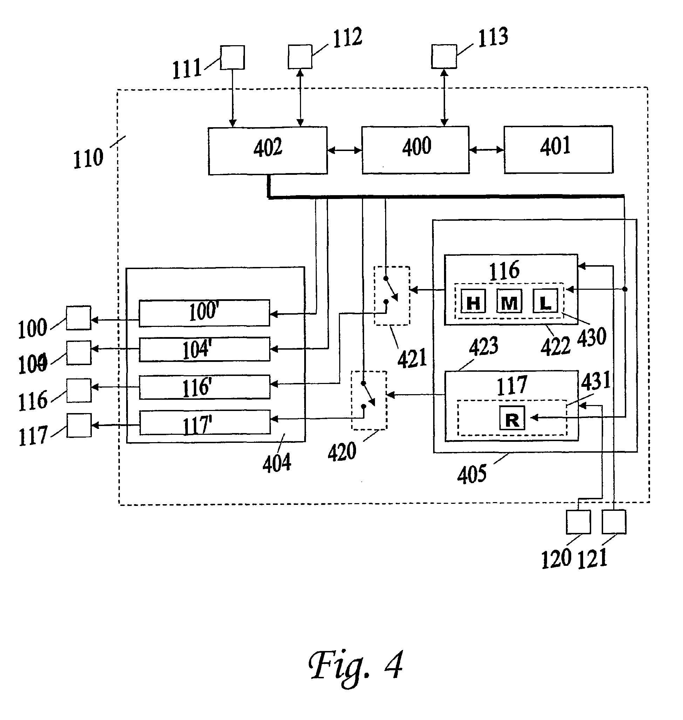

The ISL board 110 preferably comprises a controller, memory, and interface circuitries (not shown in Fig. 1), for controlling the operation of the suction pump 117, the insufflation pump 116, activation/deactivation of the lamp 103, the iris motor 104, for reading the pressure sensed by the pressure sensors 120 and 121, and for inputting and outputting data from/to display 111, the keypad and light emitting diodes (LEDs) 112, and the communication interface 113. A block diagram of a preferred embodiment of the ISL board is shown in Fig. 4 and described hereinbelow.

Insufflation pump 116 is provided with a gas input manifold 134 to allow the provision of air or other gases, such as CO₂ under pressure at the distal tip of the endoscope. A pressure relief valve 130 and a relief ("one-way") valve 132 are provided in the insufflation line since most of the DC pumps that are used to generate the positive pressure for irrigation/insulation are not able to start working against pressure. The sequence of events is as follows: the pump 116 builds up the pressure in the insufflation channel until the sensor 121 supplies a signal to the controller that will shut down the operation of the pump 116; now the pressure is reduced e.g., by user demand and than the sensor 121 signals that the pump 116 should be activated; the check valve 132 maintains the pressure in the distal part of the channel while the relief valve 130 is opened for a brief period, e.g. 10msec, to reduce the pressure in the section of the line between the pump 116 and the check valve 132 to zero; now, the pump can start to work against zero pressure and the relief valve is closed either before the pump is activated or immediately thereafter; allowing the pressure in the insufflation channel to rise.

The Lamp 103 is a white light source such as a halogen, metal halide, LED or xenon lamp, and the light source power supply should include means for operating the lamp 103. Such means are, for example, a ballast in the case of a xenon lamp, where the term "ballast" includes all the electrical components necessary to operate the lamp. Also preferably included in the power supply circuit is a case interlock to protect the eyes of the operator from the UV radiation emitted by the lamp when the console is open. The light intensity that is required depends not only the amount of light needed for a bright image at the distal tip, but also on factors such as the diameter, absorption coefficient, quality, etc of the optical fibers that are in the endoscope that is connected to the system. Additionally, the output of the lamps must be controlled to optimize the light intensity at the distal tip of the endoscope. In the preferred embodiment of the invention, the output intensity of the lamp is controlled by adjusting the opening of iris 105 to allow passage of the amount of light required for the procedure being carried out. The opening of iris 105 is adjusted by signals provided by the ISL board 110 to motor 104, which is preferably a step motor. An optical sensor may be provided to sense the home position of the motor that controls the iris. The light passing through iris 105 opening is delivered to the endoscope multi-connector 106 on the front panel of unit 150. When the connector at the proximal end of the endoscope is inserted into multi-connector 106 the light enters a light guide or optical fibers and travels through one of the endoscope's channels to the distal tip. The lamp is preferably mounted on a mechanical adjustable bracket that enables optimization of the coupling of the output of the lamp into the fiber, thereby increasing the amount of light that is gathered by the fiber and increasing the illumination at the distal end of the endoscope. Skilled persons will be aware of other arrangements to control the light intensity that can replace the iris and the motor in the ISL system. If several endoscopes are attached to the endoscopy suite, each through its own multiconnector 106, then multiple lamps, 103, ballasts, etc. may be provided in console 150. Alternatively an optical arrangement comprising one or more beam splitters can be provided to allow the light from one lamp to be used to supply light to two or more illumination channels. In either case, separate irises 105, each controlled by its own motor 104 are preferably provided to allow optimal adjustment of the light intensity for each endoscope. Other sources of light can be used illuminate the region in which the procedure is to be carried out. For example, LEDs can be placed on the distal tip of the endoscope in which case the endoscopy suite will comprise a power source connected to the LEDs by means of electrical cables.

The pressurized air supplied by insufflation pump 116 is delivered to the respective endoscope channel via the P+ connector 123 and suction created by vacuum pump 117 via the P- connector 124 on the front panel of the unit 150. The vacuum pump is part of the endoscopy suite. The pressure supplied by the insufflation pump can also be used for supplying water to the endoscope tip via a separate irrigation channel in the endoscope. This is accomplished by connecting a "Y" connector at the outlet from the P+ connector 123. One branch of the "Y" being directly connected to the proximal end of the endoscope insufflation channel and the other branch connected to the inlet of a sealed water-filled container whose outlet is connected to the proximal end of the endoscope irrigation channel. In an alternative implementation, the "Y" connector may be connected to the insufflation pump from within the console 150, where one output of the "Y" is connected to the P+ connector 123 in order to generate the irrigation and the other output of the "Y" will be connected to the insufflation channel of the endoscope through the Multi-Connector 106.

The ISL Board 110 controls the operation of insufflation pump 116 and vacuum pump 117 in accordance with the pressure sensed by the respective pressure sensors 120 and 121. The suction and insufflation pressures are maintained within the required ranges by activating the respective pump 116 or 117 whenever the pressure sensed by the respective sensor 120 or 121 deviates beyond the lower limit of proscribed conditions, and by stopping the operation of the respective pump whenever the pressure sensed by the respective sensor is above the upper limits of the proscribed conditions. For example for a Gastroscopy procedure performed with a specific endoscope, the lower limit for suction pressure is preferably limited to -0.4bar, the upper limit for suction pressure is preferably limited to -0.6bar, the low limit for insufflation pressure is preferably limited to 1.0 bar, and the upper limit for insufflation pressure is preferably limited to 1.5bar.

The keypad and LEDs 112 allow the operator to manually activate/deactivate various components of unit 150 or to select the predetermined set of parameters required for the respective procedure, and also indicate the operator's selection via the LEDs. The keypad 112 includes keys for activating and deactivating the light source and the suction and insufflation control loops. In a preferred embodiment of the invention, the operator may use keypad 112 to select a desired insufflation pressure from a set of alternatives designated e.g., low, medium, or high; wherein the exact value of pressure that corresponds to each of these alternatives has been preset according to the endoscopic procedure that will be carried out.

The ISL Board can display via the display 111 information including, but not limited to: alerts, the predetermined values of the parameters currently selected, the sensed pressures, and other information associated with the specific endoscopy procedure being carried out. The suction and insufflation pressure are preferably controlled by utilizing analog circuitries to avoid any possible software faults from interfering with the operation of pumps 116 and 117. Analog cut off circuits based on non-volatile programmable resistors (not shown) are utilized for this purpose. Implementation of such pressure control circuitries can be carried out in various ways that are well known to persons skilled in the art.

When the operator selects a specific endoscopic procedure via the keypad 112, a predetermined set of parameters appropriate to that procedure are read from the memory of the ISL board 110 and written to the respective programmable resistors. The parameters of the set preferably are values of the upper and lower recommended pressure limits for suction and insufflation. Thus, the pressure control circuitries can provide analog control means for monitoring and adjusting the insufflation and suction pressures according to the predetermined high and low bounds and establish a hysteresis loop for controlling operation of pumps 116 and 117.

The communication interface 113 is used for modifying the currently stored parameter set, i.e. calibrating the device, or for inserting new parameter sets, as may be required. However, only skilled and authorized technicians should preferably carry out these operations in order to prevent introducing unwanted and even unsafe changes in the sets of parameters stored in memory of the ISL board of unit 150.

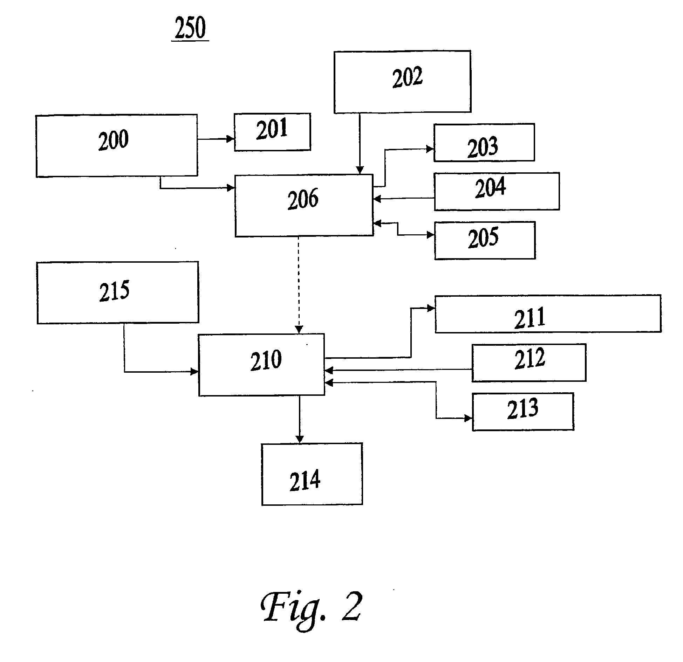

The operation of other equipment, for which specific predetermined operating parameters are not required, is preferably controlled via an independent unit (Console) 250, as exemplified in Fig. 2. In this exemplary embodiment, unit 250 comprise devices used for controlling the operation of video camera and ultrasound transducers located on the distal tip of the endoscope and connected to unit 250 via camera connector 211 and ultrasound connector 202, respectively. Unit 250 preferably comprises two power supplies 200 and 215.

The Ultrasound Power Supply 200 is used to power the ultrasound module 206 and one or more fans 201 in unit 250. Ultrasound module 206 comprises a signal generator, processing means, memory, and interfacing circuitries 205 required for emitting and acquiring ultrasound signals from the ultrasound transducer via the ultrasound connector 202. A keypad 204 is linked to the ultrasound module 206 and used by the operator to adjust various ultrasound settings (for example for calibration). The processing means provides the digital signal processing (DSP) capabilities required for processing and analyzing the acquired ultrasound signals. The results of the signal analysis are displayed on display 203, which is linked to ultrasound module 206. The ultrasound signals can be used for measuring position, distance, and tissue thickness within the body.

The video board power supply 215 powers the video module 210, the keypad 212, the keyboard 212 and the communication interface 213. The video module 210 provides the required electrical supply and control for operating the camera, which is connected to it via the camera connector 211. The image data received form the camera is processed by the video module 210 and displayed on the video display 214. To enable it to display the images, the video module 210 should include means for acquiring the image signals from the camera and processing means capable of carrying out the DSP tasks involved in processing the acquired image data. The operator can change various image properties (e.g., color intensity, brightness, zoom, etc) by means of keypad 212. The communication interface 213 can be used to connect units 150 and 250 or to connect the ultrasound module 206 to the video module 210 thus allowing data inputs to be provided from one unit to the other. In addition to displaying the images and other information acquired by the system, they can be stored in memory, transmitted to a communication network, e.g. the internet, or sent to a printer.

It should be noted that the display 203 of unit 250 is not necessarily required if the ultrasound module 206 and video module 210 are linked (broken line in Fig. 2). In that case modules 206 and 210 can be adapted to provide the information output produced by the ultrasound module 206 on the video display 214, which is directly linked to the video module 210.

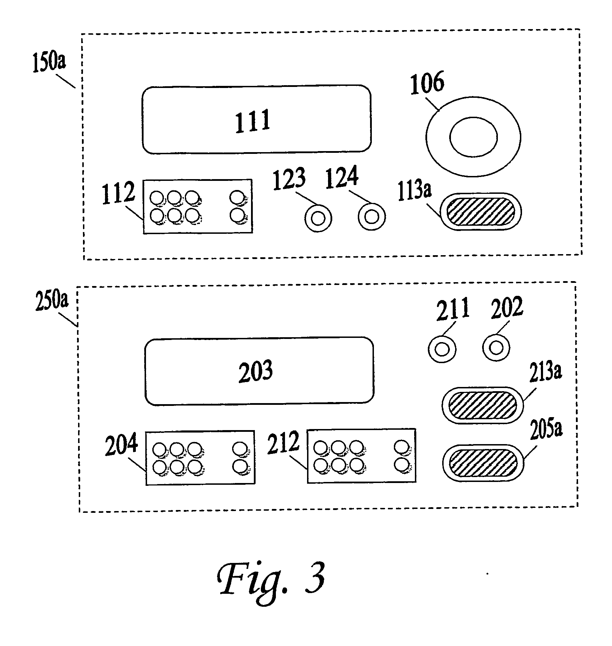

Fig. 3 schematically illustrates one embodiment of the front panels, which fulfill the function of the user interface with units 150 and 250, which together comprise the endoscopy suite. Panel 150a comprises keypad and LEDs 112, which are used to operate the ISL of unit 150, and display 111.

Panel 150a also includes the multi-connector socket 106, the P+ Connector 123, the P- Connector 124, and a communication connector 113a, which provides the external electrical connection of communication interface 113 to communication interface 213 (see Fig. 2).

Panel 250a comprises display 203, keypads 204 and 212, used to operate the video and ultrasound modules 206 and 210 of unit 250, as well as the camera connector 211, the ultrasound connector 202, and the communication connectors 213a and 205a which provides the required external electrical connections to the communication interfaces 213 and 205 respectively.

In one embodiment, the communication connectors 113a, 213a, and 205a, are located on the front panels 150a and 250a of the respective units, as shown in Fig. 3. In other embodiments they can be located on the back panels of the units. In other embodiments a keyboard can be provided in addition to or instead of keypads 112, 204, and 212. As mentioned, the endoscopy suite can be designed for simultaneous use with more than one endoscope, in which case multiple connectors and display units will be present. In preferred embodiments LEDs will be provided on the front panel to indicate operation of various subsystems of the endoscopy suite, e.g. that the illumination system is operating.

Fig. 4 is a block diagram illustrating a preferred embodiment of the ISL board 110. The instrumentation circuitries and the operation of the drivers 404 is controlled by the CPU 400 via the interface logic circuits of block 402. The CPU 400 is linked to memory 401 in which is stored the predetermined sets of parameters, the operation code, and other information. The ISL board is connected to the communication interface 113, which may simply be a suitable communication connector (e.g., RS232, IEEE 1394, USB or similar). The logic circuits in 402 provide the necessary signals for activating/deactivating the lamp, motor, insufflation pump, and vacuum pump, via the respective drivers 100', 104', 116', and 117' respectively (collectively indicated by numeral 404), in response to signals received from the CPU 400. The logic circuits in 402 are also linked to the keypad and LEDs 112 and the display 111.

The components in block 405 comprise circuitries 422 and 423 for controlling the activation/deactivation of the insufflation and vacuum pumps, 116 and 117 in response to the pressure sensed by pressure sensors 120 and 121. The cut off circuits 430 and 431 respond to the signals for activating/deactivating the pumps by activating switching devices 421 and 420. The signals from the logic circuits in 402 are connected by the switches to the respective drivers in 404 whenever an activation signal is provided by the cut off circuits 430 and 431, to the switching devices 421 and 420 respectively. In this way the activation signals provided by the logic circuits in 402 to the drivers of the insufflation and suction drivers are conditional on the signals provided by the cut off circuitries 430 and 431.

The predetermined sets of parameters stored in the memory 401 are used by the logic circuits in 402 to set the values of the non-volatile programmable resistors in the cut off circuits. The insufflation cut off circuit 430 preferably comprise six programmable resistors "H", "M", "L", for providing high, medium, and low insufflation pressure values wherein one resistor is used for the upper deactivation limit and the other is used for the lower activation limit. The suction cut off circuitry preferably comprises two programmable resistors "R", for presetting the suction pressure. This is the presently preferred embodiment however the invention can be implemented by providing more or less programmable resistors.

Although the memory 401 is shown in this example as connected only to the CPU 400, it should be noted that the logic circuits in 402 may be directly linked to memory 401 or, alternatively, to an additional memory (not shown) for providing direct access to the predetermined sets of parameters.

The endoscopy suite of the invention can be used to perform any endoscopy procedure including those selected from the group comprising: a Gastroscopy procedure; an ERCP procedure using a mother scope and a baby scope; a Colonoscopy procedure; a Gynecology procedure; a Bronchoscopy procedure; an ENT procedure; an Endoluminal anti Reflux procedure; a Transgastric or Extragastric procedure using a mother scope and one or more baby scopes; and an Anti Reflux proceedure. The Transgastric or Extragastric procedure can be selected from the group comprising: Appendectomy; Cholecystectomy; liver biopsy and excision; gastric banding for morbid obesity; and implantation of medical devices.

In order to enable the surgeon to completely control the operation with relative ease, preferred embodiments of the endoscopy suite of the invention comprise all of the control and display means necessary manipulate a number of endoscopes and also the surgical tools that must be used to perform the procedure. Thus, for example, the operation of the surgical stapler employed e.g. to close the hole in the stomach linking after a transgastric procedure can be operated and the status of the stapling procedure monitored from a single location.

The above examples and description have been provided only for the purpose of illustration, and are not intended to limit the invention in any way. As will be appreciated by the skilled person, the invention can be carried out in a great variety of ways, employing techniques different from those described above, all without exceeding the scope of the invention.

## Claims

1. An endoscopy suite comprising one or more endoscopes and:
A) an ultrasound and video unit (250) comprising:
a) ultrasound components comprising:
(i) an ultrasound power supply (200);
(ii) an ultrasound module (206) comprising an ultrasound signal generator, processing means, and interfacing circuitries (205);
(iii) an input device (204);
(iv) an ultrasound display (203);
(v) an ultrasound connector (202);
(vi) one or more fans (201);
b) video components comprising:
(i) a video board power supply (215);
(ii) a video module (210);
(iii) a video display (214);
(iv) input means (212);
(v) a camera connector (211);
(vi) a communication interface (213);
B. a suction, insufflation, and light intensity (ISL) unit (150) comprising :
(i) a light source power supply (100);
(ii) a light source (103);
(iii) means to control the output (105) of said light source (103);
(iv) cooling fans (101, 102);
(v) an endoscope multiconnector (106);
(vi) an ISL board (110) comprising controller (400) and memory means (401) and logic circuitries (402);
(vii) a power supply (115) for said ISL board (110);
(viii) an insufflation pump (116);
(ix) a suction pump (117);
(x) pressure sensors (120, 121);
(xi) valves (130, 132);
(xii) a display (111);
(xiii) input means (112); and
(xiv) a communication interface (113);
wherein:
c) said endoscopy suite comprises reconfigurable equipment and resources that are usable for carrying out different procedures at different times according to user defined or predetermined sets of parameters that are particularly suitable for the specific procedure;
d) when said operator of said endoscopy suite selects a specific endoscopic procedure via said input means (112) of said ISL unit (150), a predetermined set of parameters comprising values of the upper and lower recommended pressure limits for suction and insufflation appropriate to said procedure are read from a memory (401) of said ISL board (110) and are written to non-volatile programmable resistors in analog cut off circuits (430, 431), which are configured to control the operation of said suction means (117) and insufflation pump (116).

2. An endoscopy suite according to claim 1, wherein the light source (103) comprises one or more lamps configured to provide white light conductable to the distal end of the endoscope/s.

3. An endoscopy suite according to claim 1, wherein the light source power supply (100) includes ballast means.

4. An endoscopy suite according to claim 1, wherein the light source (103) is selected from the group comprising: xenon lamps, light emitting diodes (LEDs), halogen lamps, and metal halide lamps.

5. An endoscopy suite according to claim 1, wherein the light source (103) comprises an optical arrangement comprising one or more beam splitters, thereby allowing the light from one lamp to be used to supply light to two or more illumination channels.

6. An endoscopy suite according to claim 1, wherein the light source power supply (100) is connected by means of electrical cables to LEDs located at the distal tip of the endoscope/s.

7. An endoscopy suite according to claim 1, wherein the means to control the output (105) of the light source (103) comprises an iris (105) attached to a motor (104) adapted to respond to signals for adjusting the opening of said iris (105).

8. An endoscopy suite according to claim 7, wherein the motor (104) is a step motor.

9. An endoscopy suite according to claim 7, comprising an optical sensor to sense the home position of the iris (105).

10. An endoscopy suite according to claim 1, wherein the endoscopels comprise one or more ultrasound transducers and wherein the ultrasound module (206) is configured to generate ultrasound signals, which are emittable and receivable by said one or more ultrasound transducers on the endoscope/s; and is configured to detect and process ultrasound signals received by said transducer/s; and to output said processed signals to the ultrasound display (203).

11. An endoscopy suite according to claim 10, wherein the processed signals are used to measure one or more of the following:
- position;
- distance; and
- tissue thickness.

12. An endoscopy suite according to claim 1, wherein the input device (204) is linked to the ultrasound module (206) to allow modifying the operation and settings of said ultrasound module (206).

13. An endoscopy suite according to claim 1, wherein the endoscopels comprise one or more cameras and wherein the video module (210) is configured to acquire video signals from said one or more cameras on the one or more endoscopes, and to process said signals, and output said processed signals to one or more display devices (214), to storage devices, to a communication network, and/or to printers.

14. An endoscopy suite according to claim 1, wherein the input means (212) are linked to the video module (210) for modifying the operation and settings of said video module (210).

15. An endoscopy suite according to claim 1, wherein the video module (210) is configured to receive output signals from the ultrasound module (206) and to display said output ultrasonic signals along with the output video signals on the video display (214).

16. An endoscopy suite according to claim 7, wherein the controller (400) of the ISL board (110) is configured to manage the data stored in the memory (401), receive inputs from the input device (112), and output signals to the display (111) and to the logic circuitries (402) controlling the operation of the light source power supply (100), iris motor (104), insufflation pump (116), and suction means (117).

17. An endoscopy suite according to claim 1, comprising means for controlling and monitoring the status of surgical tools used to perform endoscopic procedures.

18. An endoscopy suite according to claim 17, wherein the surgical tool is a surgical stapler.

19. An endoscopy suite according to claim 1, wherein the endoscopy procedure is selected from the group comprising:
- a Gastroscopy procedure;
- an ERCP procedure using a mother scope and a baby scope;
- a Colonoscopy procedure;
- a Gynecology procedure;
- a Bronchoscopy procedure;
- an ENT procedure;
- a Transgastric or Extragastric procedure;
- a Transgastric or Extragastric procedure using a mother scope and one or more baby scopes; and
- an Anti Reflux procedure.

20. An endoscopy suite according to claim 19, wherein the Transgastric or Extragastric procedure is selected from the group comprising:
- Appendectomy;
- Cholecystectomy;
- liver biopsy and excision;
- Gastric banding for morbid obesity; and
- Implantation of medical devices.

## Patentansprüche

1. Endoskopieanordnung, umfassend ein oder mehrere Endoskope und:
A) eine Ultraschall- und Videoeinheit (250), umfassend:
a) Ultraschallkomponenten, umfassend:
(i) eine Ultraschall-Energieversorgung (200);
(ii) ein Ultraschall-Modul (206), umfassend einen UltraschallSignal-Erzeuger, Verarbeitungsmittel und Schnittstellenschaltkreise (205);
(iii) eine Eingabeeinrichtung (204);
(iv) eine Ultraschall-Anzeige (203);
(v) eine Ultraschall-Verbindungsvorrichung (202);
(vi) ein oder mehrere Gebläse (201);
b) Videokomponenten, umfassend:
(i) eine Videokarte-Energieversorgung (215);
(ii) ein Video-Modul (210);
(iii) eine Video-Anzeige (214);
(iv) Eingabemittel (212);
(v) eine Kamera-Verbindungsvorrichtung (211);
(vi) eine Kommunikations-Schnittstelle (213);
B. eine Absaug-, Auffüll- und Lichtintensitäts-(ISL)-Einheit (150), umfassend:
(i) eine Lichtquellen-Energieversorgung (100);
(ii) eine Lichtquelle (103);
(iii) Mittel zum Regeln/Steuern der Ausgabe (105) der Lichtquelle (103);
(iv) Kühlgebläse (101, 102);
(v) eine Endoskop-Mehrfachverbindungsvorrichtung (106);
(vi) eine ISL-Platine (110), umfassend Controller- (400) und Speichermittel (401) und Logikschaltkreise (402);
(vii) eine Energieversorgung (115) für die ISL-Platine (110);
(viii) eine Auffüllpumpe (116);
(ix) eine Absaugpumpe (117);
(x) Drucksensoren (120, 121);
(xi) Ventile (130, 132);
(xii) eine Anzeige (111);
(xiii) Eingabemittel (112); und
(xiv) eine Kommunikations-Schnittstelle (113);
wobei:
c) die Endoskopieanordnung rekonfigurierbare Ausstattung und Ressourcen umfasst, welche verwendbar sind zum Ausführen verschiedener Eingriffe zu verschiedenen Zeiten nach Maßgabe von benutzerdefinierten oder vorbestimmten Parametersätzen, die für den speziellen Eingriff besonders geeignet sind;
d) wenn der Bediener der Endoskopieanordnung einen speziellen Endoskopieeingriff über die Eingabemittel (112) der ISL-Einheit (150) auswählt, ein vorbestimmter Parametersatz, welcher Werte der unteren und oberen empfohlenen Druckgrenzen für ein Absaugen und Auffüllen umfasst, die für den Eingriff geeignet sind, aus einem Speicher (401) der ISL-Platine (110) gelesen und auf nicht-flüchtige programmierbare Widerstände in analogen Abschaltkreisen (430, 431) geschrieben wird, welche dazu eingerichtet sind, den Betrieb der Absaugmittel (117) und der Auffüllpumpe (116) zu regeln/steuern.

2. Endoskopieanordnung nach Anspruch 1, wobei die Lichtquelle (103) eine oder mehrere Lampen umfasst, welche dazu eingerichtet sind, weißes Licht bereitzustellen, das zu dem distalen Ende des Endoskops/der Endoskope leitbar ist.

3. Endoskopieanordnung nach Anspruch 1, wobei die Lichtquellen-Energieversorgung (100) Vorschaltgeräte umfasst.

4. Endoskopieanordnung nach Anspruch 1, wobei die Lichtquelle (103) ausgewählt ist aus der Gruppe, umfassend: Xenonlampen, lichtemittierende Dioden (LEDs), Halogenlampen und Halogen-Metalldampflampen.

5. Endoskopieanordnung nach Anspruch 1, wobei die Lichtquelle (103) eine optische Anordnung umfasst, umfassend einen oder mehrere Strahlteiler, wodurch es dem Licht einer Lampe möglich ist, dazu verwendet zu werden, zwei oder mehr Beleuchtungskanälen Licht zu liefern.

6. Endoskopieanordnung nach Anspruch 1, wobei die Lichtquellen-Energieversorgung (100) mittels elektrischer Kabel mit LEDs verbunden ist, welche an der distalen Spitze des Endoskops/der Endoskope angeordnet sind.

7. Endoskopieanordnung nach Anspruch 1, wobei die Mittel zum Regeln/Steuern der Ausgabe (105) der Lichtquelle (103) eine Blende (105) umfassen, die an einem Motor (104) angebracht ist, welcher dazu eingerichtet ist, auf Signale zum Einstellen der Öffnung der Blende (105) zu reagieren.

8. Endoskopieanordnung nach Anspruch 7, wobei der Motor (104) ein Schrittmotor ist.

9. Endoskopieanordnung nach Anspruch 7, umfassend einen optischen Sensor, um die Ausgangsposition der Blende (105) zu erkennen.

10. Endoskopieanordnung nach Anspruch 1, wobei das Endoskop/die Endoskope einen oder mehrere Ultraschall-Wandler umfassen und wobei das Ultraschall-Modul (206) dazu eingerichtet ist, Ultraschall-Signale zu erzeugen, welche von dem einen oder den mehreren Ultraschall-Wandlern an dem Endoskop/den Endoskopen emittierbar und empfangbar sind; und dazu eingerichtet ist, von dem Wandler/den Wandlern empfangene Ultraschall-Signale zu detektieren und zu verarbeiten; und die verarbeiteten Signale an die Ultraschall-Anzeige (203) auszugeben.

11. Endoskopieanordnung nach Anspruch 10, wobei die verarbeiteten Signale dazu verwendet werden, eines oder mehrere der folgenden zu messen:
- Position;
- Abstand; und
- Gewebestärke.

12. Endoskopieanordnung nach Anspruch 1, wobei die Eingabevorrichtung (204) mit dem Ultraschall-Modul (206) verbunden ist, um ein Modifizieren des Betriebs und der Einstellungen des Ultraschall-Moduls (206) zu gestatten.

13. Endoskopieanordnung nach Anspruch 1, wobei das Endoskop/die Endoskope eine oder mehrere Kameras umfassen und wobei das Video-Modul (210) dazu eingerichtet ist, Video-Signale von der einen oder den mehreren Kameras an dem einen oder den mehreren Endoskopen zu erhalten und die Signale zu verarbeiten, und die verarbeiteten Signale an ein oder mehrere Anzeigevorrichtungen (214), an Speichervorrichtungen, an ein Kommunikations-Netzwerk oder/und an Drucker auszugeben.

14. Endoskopieanordnung nach Anspruch 1, wobei die Eingabemittel (212) mit dem Video-Modul (210) verbunden sind, um den Betrieb und die Einstellungen des Video-Moduls (210) zu modifizieren.

15. Endoskopieanordnung nach Anspruch 1, wobei das Video-Modul (210) dazu eingerichtet ist, Ausgabesignale von dem Ultraschall-Modul (206) zu empfangen und die ausgegebenen Ultraschall-Signale zusammen mit den ausgegebenen Video-Signalen auf der Video-Anzeige (214) anzuzeigen.

16. Endoskopieanordnung nach Anspruch 7, wobei der Controller (400) der ISL-Platine (110) dazu eingerichtet ist, die in dem Speicher (401) gespeicherten Daten zu verwalten, Eingaben von der Eingabevorrichtung (112) zu empfangen und Signale an die Anzeige (111) und an die Logik-Schaltkreise (402) auszugeben, welche den Betrieb der Lichtquellen-Energieversorgung (100), des Blendenmotors (104), der Auffüllpumpe (116) und der Absaugmittel (117) regeln/steuern.

17. Endoskopieanordnung nach Anspruch 1, umfassend Mittel zum Regeln/Steuern und Überwachen des Status von chirurgischen Werkzeugen, welche zum Ausführen endoskopischer Eingriffe verwendet werden.

18. Endoskopieanordnung nach Anspruch 17, wobei das chirurgische Werkzeug ein chirurgischer Tacker ist.

19. Endoskopieanordnung nach Anspruch 1, wobei der endoskopische Eingriff ausgewählt ist aus der Gruppe, umfassend:
- Gastroskopie-Eingriff;
- ein ERCP-Eingriff unter Verwendung eines Mutterskops und eines Babyskops;
- ein Koloskopie-Eingriff;
- ein Gynäkologie-Eingriff;
- ein Bronchoskopie-Eingriff;
- ein ENT-Eingriff;
- ein transgastrischer oder extragastrischer Eingriff;
- ein transgastrischer oder extragastrischer Eingriff unter Verwendung eines Mutterskops und eines oder mehrerer Babyskope; und
- ein Antireflux-Eingriff.

20. Endoskopieanordnung nach Anspruch 19, wobei der transgastrische oder extragastrische Eingriff ausgewählt ist aus der Gruppe, umfassend:
- Appendektomie;
- Cholezystektomie;
- Leberbiopsie und -exzision;
- Magenband für morbide Adipositas; und
- Implantation von medizinischen Vorrichtungen.

## Revendications

1. Equipement pour endoscopie comprenant un ou plusieurs endoscope(s) et:
A) une unité à ultrasons et vidéo (250) comprenant:
a) des composants ultrasoniques comprenant:
(i) une alimentation électrique pour les ultrasons (200);
(ii) un module ultrasonique (206) comprenant un générateur de signal ultrasonique, des moyens de traitement, et des circuits d'interface (205);
(iii) un dispositif d'entrée (204);
(iv) un écran d'affichage ultrasonique (203);
(v) un connecteur à ultrason (202);
(vi) un ou plusieurs ventilateurs (201);
b) des composants vidéo comprenant:
(i) une alimentation électrique de carte vidéo (215);
(ii) un module vidéo (210);
(iii) un écran d'affichage vidéo (214);
(iv) des moyens d'entrée (212);
(v) un connecteur de caméra (211);
(vi) une interface de communication (213);
B) une unité d'aspiration, d'insufflation et d'intensité lumineuse (ISL) (150), comprenant:
(i) une alimentation électrique de source lumineuse (100);
(ii) une source lumineuse (103);
(iii) des moyens de commande de la sortie (105) de ladite source lumineuse (103);
(iv) des ventilateurs de refroidissement (101, 102);
(v) un multi-connecteur d'endoscope (106);
(vi) une carte ISL (110) comprenant un contrôleur (400) et des moyens de mémoire (401) et des circuits logiques (402);
(vii) une alimentation électrique (115) pour ladite carte ISL (110);
(viii)une pompe d'insufflation (116);
(ix) une pompe d'aspiration (117);
(x) des capteurs de pression (120, 121);
(xi) des soupapes (130, 132);
(xii) un écran d'affichage (111);
(xiii)des moyens d'entrée (112); et
(xiv) une interface de communication (113);
dans lequel
c) ledit équipement pour endoscopie comprend un équipement reconfigurable et des ressources qui sont utilisables pour exécuter différentes procédures à différents instants selon des ensembles de paramètres définis ou prédéterminés par un utilisateur, qui sont particulièrement appropriés pour la procédure spécifique;
d) lorsque ledit opérateur dudit équipement pour endoscopie sélectionne une procédure endoscopique spécifique via lesdits moyens d'entrée (112) de ladite unité ISL (150), un ensemble prédéterminé de paramètres comprenant des valeurs des limites de pression supérieures et inférieures recommandées pour l'aspiration et l'insufflation appropriées pour ladite procédure sont lues à partir d'une mémoire (401) de ladite carte ISL (110) et sont écrites dans des résistances programmables non volatiles dans des circuits de coupure analogiques (430, 431), qui sont configurés pour commander le fonctionnement desdits moyens d'aspiration (117) et de la pompe d'insufflation (116).

2. Equipement pour endoscopie selon la revendication 1, dans lequel la source lumineuse (103) comprend une ou plusieurs lampe(s) configurée(s) pour fournir une lumière blanche pouvant être conduite à l'extrémité distale de l'endoscope/des endoscope(s).

3. Equipement pour endoscopie selon la revendication 1, dans lequel l'alimentation électrique de la source lumineuse (100) comprend des moyens de ballast.

4. Equipement pour endoscopie selon la revendication 1, dans lequel la source lumineuse (103) est sélectionnée dans le groupe comprenant des lampes au xénon, des diodes électroluminescentes (DEL), des lampes à halogène, et des lampes à halogénure métallique.

5. Equipement pour endoscopie selon la revendication 1, dans lequel la source lumineuse (103) comprend un agencement optique comportant un ou plusieurs diviseur(s) de faisceau, permettant ainsi à la lumière d'une lampe d'être utilisée pour fournir de la lumière à deux ou plusieurs canaux d'éclairage.

6. Equipement pour endoscopie selon la revendication 1, dans lequel l'alimentation électrique (100) de la source lumineuse est connectée au moyen de câbles électrique à des DEL situées à l'extrémité distale de l'endoscope/des endoscope(s).

7. Equipement pour endoscopie selon la revendication 1, dans lequel les moyens pour commander la sortie (105) de la source lumineuse (103) comprennent un iris (105) attaché à un moteur (104) apte à réagir à des signaux pour ajuster l'ouverture dudit iris (105).

8. Equipement pour endoscopie selon la revendication 7, dans lequel le moteur (104) est un moteur pas à pas.

9. Equipement pour endoscopie selon la revendication 7, comprenant un capteur optique pour détecter la position de repos de l'iris (105).

10. Equipement pour endoscopie selon la revendication 1, dans lequel l'endoscope/les endoscope(s) comprend/comprennent un ou plusieurs transducteur(s) ultrasonique(s) et dans lequel le module ultrasonique (206) est configuré pour générer des signaux ultrasoniques, qui peuvent être émis et reçus par ledit/lesdits un ou plusieurs transducteur(s) ultrasonique(s) sur l'endoscope/les endoscopes, et est configuré pour détecter et traiter des signaux ultrasoniques reçus par ledit/lesdits transducteur(s), et pour envoyer lesdits signaux traités à l'écran d'affichage ultrasonique (203).

11. Equipement pour endoscopie selon la revendication 10, dans lequel les signaux traités sont utilisés pour mesurer une ou plusieurs des grandeurs suivantes:
- position;
- distance; et
- épaisseur du tissu.

12. Equipement pour endoscopie selon la revendication 1, dans lequel le dispositif d'entrée (204) est relié au module ultrasonique (206) afin de permettre de modifier le fonctionnement et les réglages dudit module ultrasonique (206).

13. Equipement pour endoscopie selon la revendication 1, dans lequel l'endoscope/les endoscopes comprend /comprennent une ou plusieurs caméra(s) et dans lequel le module vidéo (210) est configuré pour acquérir des signaux vidéo sur ladite/lesdites une ou plusieurs caméra(s) sur ledit/lesdits un ou plusieurs endoscope(s), et pour traiter lesdits signaux et envoyer lesdits signaux traités à un ou plusieurs dispositif(s) d'affichage (214), à des dispositifs de stockage, à un réseau de communication, et/ou à des imprimantes.

14. Equipement pour endoscopie selon la revendication 1, dans lequel les moyens d'entrée (212) sont reliés au module vidéo (210) pour modifier le fonctionnement et les réglages dudit module vidéo (210).

15. Equipement pour endoscopie selon la revendication 1, dans lequel le module vidéo (210) est configuré pour recevoir des signaux de sortie en provenance du module ultrasonique (206) et pour afficher lesdits signaux ultrasoniques de sortie en même temps que les signaux vidéo de sortie sur l'écran d'affichage vidéo (214).

16. Equipement pour endoscopie selon la revendication 7, dans lequel le contrôleur (400) de la carte ISL (110) est configuré pour gérer les données stockées dans la mémoire (401), recevoir des entrées en provenance du dispositif d'entrée (112), et envoyer des signaux à l'écran d'affichage (111) et aux circuits logiques (402) commandant le fonctionnement de l'alimentation électrique (100) de la source lumineuse, du moteur de l'iris (104), de la pompe d'insufflation (116) et des moyens d'aspiration (117).

17. Equipement pour endoscopie selon la revendication 1, comprenant des moyens pour commander et surveiller l'état d'instruments chirurgicaux utilisés pour exécuter des procédures endoscopiques.

18. Equipement pour endoscopie selon la revendication 17, dans lequel l'instrument chirurgical est une agrafeuse chirurgicale.

19. Equipement pour endoscopie selon la revendication 1, dans lequel la procédure endoscopique est sélectionnée dans le groupe comprenant:
- une procédure de gastroscopie;
- une procédure CPRE utilisant un endoscope mère et un endoscope bébé;
- une procédure de colonoscopie;
- une procédure de gynécologie;
- une procédure de bronchoscopie;
- une procédure ORL;
- une procédure transgastrique ou extragastrique;
- une procédure transgastrique ou extragastrique utilisant un endoscope mère et un ou plusieurs endoscope(s) bébé(s); et
- une procédure anti-reflux.

20. Equipement pour endoscopie selon la revendication 19, dans lequel la procédure transgastrique ou extragastrique est sélectionnée dans le groupe comprenant:
- appendicectomie;
- cholécystectomie;
- biopsie et excision du foie;
- pose d'un anneau gastrique pour obésité morbide; et
- implantation de dispositifs médicaux.
